(19) Europäisches Patentamt
European Patent Office
Office européen des brevets

(11) **EP 1 989 304 B1**

(12) **EUROPEAN PATENT SPECIFICATION**

(45) Date of publication and mention
of the grant of the patent:
**05.05.2010 Bulletin 2010/18**

(51) Int Cl.:
*C12N 9/64* (2006.01)    *C07K 14/745* (2006.01)
*C07K 14/78* (2006.01)    *A61K 38/43* (2006.01)
*A61K 38/36* (2006.01)    *A61K 38/39* (2006.01)

(21) Application number: **07726469.5**

(22) Date of filing: **22.02.2007**

(86) International application number:
**PCT/EP2007/051711**

(87) International publication number:
**WO 2007/096406 (30.08.2007 Gazette 2007/35)**

(54) **A METHOD OF REMOVING A NON-METALLIC PROTEIN INHIBITOR FROM A LIQUID PHARMACEUTICAL PREPARATION**

VERFAHREN ZUR ENTFERNUNG EINES NICHTMETALLISCHEN PROTEINHEMMERS AUS EINER FLÜSSIGEN PHARMAZEUTISCHEN ZUBEREITUNG

MÉTHODE D'ÉLIMINATION D'UN INHIBITEUR NON MÉTALLIQUE DE PROTÉINE D'UNE PRÉPARATION PHARMACEUTIQUE LIQUIDE

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.02.2006 EP 06110412**

(43) Date of publication of application:
**12.11.2008 Bulletin 2008/46**

(73) Proprietor: **Novo Nordisk Health Care AG
8050 Zürich (CH)**

(72) Inventors:
• **JENSEN, Michael, Bech
DK-3450 Allerød (DK)**
• **NØSTED, Ulrik
DK-3520 Farum (DK)**
• **PILGAARD, Karen
DK-2860 Søborg (DK)**

(74) Representative: **Thorsen, Jesper et al
Inspicos A/S
Kogle Allé 2
P.O. Box 45
2970 Hørsholm (DK)**

(56) References cited:
**EP-A- 0 229 026     WO-A-93/01309
WO-A-97/15391**

• **HEIJBEL A ET AL.: "Removal and purification of trypsin-like serine proteases" FASEB JOURNAL, vol. 17, no. 4-5, March 2003 (2003-03), pages A999-A1000, XP008066024 ISSN: 0892-6638**
• **BAJAJ SP ET AL.: "A PROCEDURE FOR ISOLATION OF HUMAN PROTEIN C AND PROTEIN S AS BY-PRODUCTS OF THE PURIFICATION OF FACTORS VII, IX, X AND PROTHROMBIN" PREPARATIVE BIOCHEMISTRY, vol. 13, no. 3, 1983, pages 191-214, XP008066019**

**Description**

FIELD OF THE INVENTION

The present invention relates to the field of liquid pharmaceutical preparations comprising therapeutically active proteins, and in particular to the removal of non-metallic protein inhibitors from such preparations.

BACKGROUND OF THE INVENTION

Pharmaceutical preparations based on sensitive proteins are often formulated as freeze-dried products. Mostly, such pharmaceutical protein formulations are intended to be administered in the form of injections. Before injection, the freeze-dried product has to be prepared as a solution and this reconstitution step is characterised by being time-consuming, complicated and introduces risk of contamination of the final solution.

To avoid this, it is desirable to supply pharmaceutical formulations intended for injection as solutions ready for immediate use.

In the case of pharmaceutical solutions based on activated proteins, like Factor VIIa, a stabiliser in the form of an inhibitor for the activated protein is typically needed in order to prevent auto-degradation of the active protein during storage. However, some stabilisers may cause potential undesirable side-effects and the concentration in the pharmaceutical solution should therefore be as low as possible.

The binding of serine protease inhibitors like benzamidine to trypsin is described (Eur. J. Biochem. 268, 1554-1560 (2001)), and amidinophenylureas-based inhibitors (Bioorg. Med. Chem. Lett. 13, 1463-1467 (2003)) is described to stabilise the serine protease Factor VIIa against auto-catalytic degradation or -inactivation. Amidine types of stabilisers may show undesirable side-effects and may act as anti-coagulants. In the case of Factor VIIa solutions, any anticoagulant effect of the inhibitors is certainly not desirable as it would counter the effect of the active pharmaceutical ingredient.

Thus, on the one hand, and for general safety reasons, it would therefore be advantageous to reduce the concentration to an appropriate low level, but on the other hand, the level of stabiliser needed in order to stabilize the protein is typically in the concentration range of 0.05 to 50 mM, of course depending on the inhibitor and FVIIa concentration used.

Hence, there is a need for means for the preparation of ready-to-use protein solutions having a negligible content of protein inhibitor.

WO 97/15391 A discloses the use of zeolites for removing preservatives for a polypeptide solution with a reduction of the preservative level 10-99% of the original concentration without any significant effect on the polypeptide.

SUMMARY OF THE INVENTION

In view of the above-stated needs, the present invention provides a method of removing a protein inhibitor from a liquid pharmaceutical preparation, said method comprising the steps of:

(a) providing a sealed container comprising an initial liquid pharmaceutical preparation comprising (i) a protein belonging to the group of serine proteases/Vitamin K-dependent proteins and (ii) a non-metallic inhibitor for said protein;

(b) contacting said pharmaceutical preparation with a solid phase polymeric material capable of retaining at least a substantial portion of said inhibitor; and

(c) separating said liquid pharmaceutical preparation from said solid phase polymeric material so as to obtain a resulting liquid pharmaceutical preparation having a reduced concentration of said inhibitor compared to the initial liquid pharmaceutical preparation.

BRIEF DESCRIPTION OF THE FIGURES

Figure 1 shows the removal of S-2-[3-(4-carbamimidoylphenyl)-ureido]-N-[1-(3-methoxyphenyl)-ethyl]-acetamide by Chelex 100.

Figure 2 shows the removal of p-amino-benzamidine by Chelex 100.

Figure 3 shows a syringe or other compartment wherein the polymeric material is situated in the outlet zone. During injection the liquid formulation passes through the polymeric solid material and the inhibitor are removed.

Figure 4a and 4b show a syringe or other compartment wherein a sterile filter holding the polymeric material is connected with the intravenous injection tube and a needle, e.g. a butterfly needle, is mounted on the outlet. During injection the liquid formulation passes through the polymeric solid material and the inhibitor are removed.

Figure 5 shows an injection tube for mounting on a syringe or other compartment, wherein the polymeric material is either coated on the inside or filled in the tube; the tube is mounted with a needle, e.g. a butterfly needle. During injection the liquid formulation passes through the polymeric solid material and the inhibitor are removed.

Figure 6 shows removal of inhibitor 2-[3-(4-Carbamimidoylphenyl)-ureido]-N-[1-(3-methoxyphenyl)-ethyl]-acetamide from a rFVIIa formulation by Chelex 100.

## DETAILED DESCRIPTION OF THE INVENTION

As mentioned above, the present invention, i.a., provides a method of removing a protein inhibitor from a liquid pharmaceutical preparation, said method comprising the steps of:

(a) providing a sealed container comprising an initial liquid pharmaceutical preparation comprising (i) a protein belonging to the group of serine proteases/Vitamin K-dependent proteins and (ii) a non-metallic inhibitor for said protein;

(b) contacting said pharmaceutical preparation with a solid phase polymeric material capable of retaining at least a substantial portion of said inhibitor; and

(c) separating said liquid pharmaceutical preparation from said solid phase polymeric material so as to obtain a resulting liquid pharmaceutical preparation having a reduced concentration of said inhibitor compared to the initial liquid pharmaceutical preparation.

The invention is based on the finding that the amount of non-metallic inhibitors for particular proteins belonging to the group of serine proteases/Vitamin K-dependent proteins in liquid pharmaceutical preparations of such therapeutically relevant proteins can efficiently be reduced or virtually eliminated by means of contacting the solution to a solid phase polymeric material for a relatively short period of time and in such a manner that virtually no loss of the therapeutically active protein or the activity of the therapeutically active protein is observed.

*Method step (a)*

In the first step of the method, a sealed container comprising an initial liquid pharmaceutical preparation comprising (i) a protein belonging to the group of serine proteases/Vitamin K-dependent proteins and (ii) a non-metallic inhibitor for said protein is provided.

The term "initial liquid pharmaceutical preparation" is intended to mean a liquid pharmaceutical composition (typically an aqueous pharmaceutical preparation) which is formulated as a ready-to-use (typically ready-to-inject) composition, except for the presence of an undesirable high amount of the inhibitor. Preparation of such liquid pharmaceutical preparations is known by the person skilled in the art, e.g. from WO 2005/016365 A.

The liquid preparation is typically isotonic or hypertonic. The term "isotonic" means "isotonic with serum", i.e. at about $300 \pm 50$ milliosmol/kg. The tonicity is meant to be a measure of osmolality of the solution prior to administration. The term "hypertonic" is meant to designate levels of osmolality above the physiological level of serum, such as levels above $300 \pm 50$ milliosmol/kg (isotonic).

The preparation comprises, as mandatory components, (i) a protein belonging to the group of serine proteases/Vitamin K-dependent proteins and (ii) a non-metallic inhibitor for said protein.

Serine proteases/Vitamin K-dependent proteins

It is to be understood that the invention especially relevant for proteins having auto-degrading catalytic properties, thus, in particular for where the protein is in partly or fully activated form.

Moreover, the invention is particularly beneficial for protein belonging to the group of serine proteases/Vitamin K-dependent plasma proteins.

The term "vitamin K-dependent protein", as used herein, means any protein that is gamma-carboxylated on glutamic acid residues. Vitamin-K dependent coagulation factors require gamma-carboxylation of the Gla-domain for full activity. Gamma-carboxylated glutamic acid (Gla) is an amino acid found in certain calcium-binding proteins. Typical vitamin K-dependent proteins includes but are not limited to the procoagulant clotting factors prothrombin (factor II), factor VII, factor IX, factor X; the anticoagulants Protein C and Protein S; Protein Z, also found in plasma; pulmonary surfactant-associated proteins (Rannels et al. Proc. Natl. Acad. Sci. USA 84: 5952-56, 1987), and the bone proteins osteocalcin (also known as bone Gla-protein), proline-rich Gla protein 1, and matrix Gla-protein. Proteins containing this amino acid are variously referred to as "Vitamin K-dependent proteins", "Gla-proteins", or "gamma-carboxylated proteins."

The biosynthesis of vitamin K-dependent proteins includes several post-translational processing steps before a mature functional protein is obtained. Vitamin K is a necessary cofactor for the gamma-carboxylation of glutamic acid residues in these vitamin K-dependent proteins. The gamma-carboxylated glutamic acid (Gla) residues are required for the metal-associated interaction of these proteins with membrane phospholipids. Gamma-carboxylation permits the coagulation proteins to undergo a conformational change necessary both for calcium-dependent complexing of vitamin K-dependent proteins to their cofactors on phospholipid surfaces and for their biologic activity.

Suitable examples of such proteases/proteins are those selected from the group consisting of GAS-6, Protein S, Factor II (Prothrombin), Factor X polypeptides, Factor IX polypeptides, Protein C, Factor VII polypeptides, Protein Z, Transmembrane gamma-carboxyglutamic acid protein 1, Transmembrane gamma-carboxyglutamic acid protein 2, Transmembrane gamma carboxyglutamic acid protein 3, Transmembrane gamma-carboxyglutamic acid protein 4, Matrix Gla protein, and Osteocalcin, in particular Vitamin K-dependent coagulation factors selected from Factor VII polypeptides, Factor IX polypeptides, Factor X polypeptides and activated Protein C. It should be understood that PEGylated derivatives, truncated forms, and sequence variants of the before-mentioned proteins are also encompassed so long as those proteins retain at least 10% of the biological activity of the respective parent polypeptide.

The term "variants", as used herein, is intended to designate a serine protease/vitamin K-dependent protein, e.g. FVII, wherein one or more amino acid residues of the parent protein have been substituted by another amino acid residue and/or wherein one or more amino acid residues of the parent protein have been deleted and/or wherein one or more amino acid residues have been added to the parent protein. Such addition can take place either at the N-terminal end or at the C-terminal end of the parent protein or both. It can also take place within the amino acid chain of the parent protein ("in-chain" addition), optionally at the same time as addition(s) at the C-terminal and/or N-terminal of the parent protein.

Such proteases/proteins may be obtained in various ways, e.g. by isolation from natural sources such as tissue, blood or other body fluids, or by recombinant techniques, e.g. production in transgenic animals (e.g. fish, rabbit, pig, cow) or by production under cell culture conditions.

In some preferred embodiments, the protein is a Vitamin K-dependent coagulation factor selected from the group consisting of Factor VII polypeptides, Factor IX polypeptides, Factor X polypeptides and activated Protein C.

As used herein, the terms "Factor VII polypeptide " or "FVII polypeptide" means any protein comprising the amino acid sequence 1-406 of wild-type human Factor VIIa (i.e., a polypeptide having the amino acid sequence disclosed in U.S. Patent No. 4,784,950), variants, thereof as well as Factor VII-related polypeptides, Factor VII derivatives and Factor VII conjugates. This includes FVII variants, Factor VII-related polypeptides, Factor VII derivatives and Factor VII conjugates exhibiting substantially the same or improved biological activity relative to wild-type human Factor VIIa.

The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like.

"Factor VII" or "Factor VIIa" within the above definition also includes natural allelic variations that may exist and occur from one individual to another. Also, degree and location of glycosylation or other post-translation modifications may vary depending on the chosen host cells and the nature of the host cellular environment.
As used herein, "wild type human FVIIa" is a polypeptide having the amino acid sequence disclosed in U.S. Patent No. 4,784,950.

As used herein, "Factor VII-related polypeptides" encompasses polypeptides, including variants, in which the Factor VIIa biological activity has been substantially modified, such as reduced, relative to the activity of wild-type Factor VIIa. These polypeptides include, without limitation, Factor VII or Factor VIIa into which specific amino acid sequence alterations have been introduced that modify or disrupt the bioactivity of the polypeptide.

The term "Factor VII derivative" as used herein, is intended to designate a FVII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, acylation, ester formation or amide formation, fusion with a polypeptide (including albumin-fusion proteins as described in WO 01//79271), or the like. This includes but is not limited to PEGylated human Factor VIIa, cysteine-PEGylated human Factor VIIa, glycopegylated human Factor VIIa, and variants thereof. The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide ("GlycoPegylated Factor VIIa"). The term "cysteine-PEGylated human Factor VIIa" means Factor VIIa having a PEG molecule conjugated to a sulfhydryl group of a cysteine introduced in human Factor VIIa. Non-limiting examples of Factor VII derivatives includes GlycoPegylated FVII derivatives as disclosed in WO 03/31464 and US Patent applications US 20040043446, US 20040063911, US 20040142856, US 20040137557, and US 20040132640 (Neose Technologies, Inc.); FVII conjugates as disclosed in WO 01/04287, US patent application 20030165996, WO 01/58935, WO 03/93465 (Maxygen ApS) and WO 02/02764, US patent application 20030211094 (University of Minnesota).

The term "improved biological activity" refers to FVII polypeptides with i) substantially the same or increased proteolytic activity compared to recombinant wild type human Factor VIIa or ii) to FVII polypeptides with substantially the same or increased TF binding activity compared to recombinant wild type human Factor VIIa or iii) to FVII polypeptides with substantially the same or increased half life in blood plasma compared to recombinant wild type human Factor VIIa.

Non-limiting examples of Factor VII variants having substantially the same or increased proteolytic activity compared to recombinant wild type human Factor VIIa include S52A-FVIIa, S60A-FVIIa ( Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189 (corresponding to WO 02/077218); and FVII variants exhibiting increased proteolytic stability as disclosed in WO 02/38162 (Scripps Research Institute); FVII variants having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and FVII variants as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of FVII variants having increased biological activity compared to wild-type FVIIa include FVII variants as disclosed in WO 01/83725, WO 02/22776, WO 02/077218, PCT/DK02/00635 (corresponding to WO 03/027147), Danish patent application PA 2002 01423 (corresponding to WO 04/029090), Danish patent application PA 2001 01627 (corresponding to WO 03/027147), WO 05/24006, WO 05/123916; WO 02/38162 (Scripps Research Institute); and FVIIa variants with enhanced activity as disclosed in JP 2001061479 (Chemo-Sero-Therapeutic Res Inst.).

Examples of variants of factor VII include, without limitation, P10Q-FVII, K32E-FVII, P10Q/K32E-FVII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/MZ98Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V158T/E296V/M298Q-FVII, S314E/L305V/V158T/K337A/M298Q-FVII, S314E/L305V/V158T/E296V/K337A-FVII, S314E/L305V/V158D/K337A/M298Q-FVII, S314E/L305V/V158D/E296V/K337A-FVII, S314E/L305V/V158D/E296V/M298Q/K337A-FVII, S314E/L305V/V158T/E296V/M298Q/K337A-FVII, K316H/L305V/K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305V/V158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A -FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/MZ98Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A-FVII, K316Q/L305V/V158D/E296V/M298Q/K337A-FVII, K316Q/L305V/V158T/E296V/M298Q/K337A-FVII, F374Y/K337A-FVII, F374Y/V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/L305V/V158D-FVII, F374Y/L305V/E296V-FVII, F374Y/L305V/M298Q-FVII, F374Y/L305V/V158T-FVII, F374Y/L305V/S314E-FVII, F374Y/K337A/S314E-FVII, F374Y/K337A/V158T-FVII, F374Y/K337A/M298Q-FVII, F374Y/K337A/E296V-FVII, F374Y/K337A/V158D-FVII, F374Y/V158D/S314E-FVII, F374Y/V158D/M298Q-FVII, F374Y/V158D/E296V-FVII, F374Y/V158T/S314E-FVII, F374Y/V158T/M298Q-FVII, F374Y/V158T/E296V-FVII," F374Y/E296V/S314E-FVII, F374Y/S314E/M298Q-FVII, F374Y/E296V/M298Q-FVII, F374Y/L305V/K337A/V158D-FVII, F374Y/L305V/K337A/E296V-FVII, F374Y/L305V/K337A/M298Q-FVII, F374Y/L305V/K337A/V158T-FVII, F374Y/L305V/K337A/S314E-FVII, F374Y/L305V/V158D/E296V-FVII, F374Y/L305V/V158D/M298Q-FVII, F374Y/L305V/V158D/S314E-FVII, F374Y/L305V/E296V/M298Q-FVII, F374Y/L305V/E296V/V158T-FVII, F374Y/L305V/E296V/S314E-FVII, F374Y/L305V/M298Q/V158T-FVII, F374Y/L305V/M298Q/S314E-FVII, F374Y/L305V/V158T/S314E-FVII, F374Y/K337A/S314E/V158T-FVII, F374Y/K337A/S314E/M298Q-FVII, F374Y/K337A/S314E/E296V-FVII, F374Y/K337A/S314E/V158D-FVII, F374Y/K337A/V158T/MZ98Q-FVII, F374Y/K337A/V158T/E296V-FVII, F374Y/K337A/M298Q/E296V-FVII, F374Y/K337A/M298Q/V158D-FVII, F374Y/K337A/E296V/V158D-FVII, F374Y/V158D/S314E/M298Q-FVII, F374Y/V158D/S314E/E296V-FVII, F374Y/V158D/M298Q/E296V-FVII, F374Y/V158T/S314E/E296V-FVII, F374Y/V158T/S314E/M298Q-FVII, F37.4Y/V158T/M298Q/E296V-FVII, F374Y/E296V/S314E/M298Q-FVII, F374Y/L305V/M298Q/K337A/S314E-FVII, F374Y/L305V/E296V/K337A/S314E-FVII, F374Y/E296V/M298Q/K337A/S314E-FVII, F374Y/L305V/E296V/M298Q/K337A -FVII, F374Y/L305V/E296V/M298Q/S314E-FVII, F374Y/V158D/E296V/M298Q/K337A-FVII, F374Y/V158D/E296V/M298Q/S314E-FVII, F374Y/L305V/V158D/K337A/S314E-FVII, F374Y/V158D/M298Q/K337A/S314E-FVII, F374Y/V158D/ E296V/K337A/S314E-FVII, F374Y/L305V/V158D/EZ96V/M298Q-FVII, F374Y/L305V/V1S8D/M298Q/K337A-FVII, F374Y/L305V/V158D/E296V/K337A-FVII, F374Y/L305V/V158D/M298Q/S314E-FVII, F374Y/L305V/V158D/E296V/S314E-FVII, F374Y/V158T/E296V/M298Q/K337A-FVII, F374Y/V158T/E296V/M298Q/S314E-FVII, F374Y/L305V/V158T/K337A/S314E-FVII, F374Y/V158T/M298Q/K337A/S314E-FVII, F374Y/V158T/E296V/K337A/S314E-FVII, F374Y/L305V/V158T/E296V/M298Q-FVII, F374Y/L305V/V158T/M298Q/K337A-FVII, F374Y/L305V/V158T/E296V/K337A-FVII, F374Y/L305V/V158T/M298Q/S314E-FVII, F374Y/L305V/V158T/E296V/S314E-FVII, F374Y/E296V/M298Q/K337A/V158T/S314E-FVII, F374Y/V158D/E296V/M298Q/K337A/S314E-FVII, F374Y/L305V/V158D/E296V/M298Q/S314E-FVII, F374Y/L305V/E296V/M298Q/V158T/S314E-FVII, F374Y/L305V/E296V/M298Q/K337A/V158T-FVII, F374Y/L305V/E296V/K337A/V158T/S314E-FVII, F374Y/L305V/M298Q/K337A/V158T/S314E-FVII, F374Y/L305V/V158D/E296V/M298Q/K337A-FVII, F374Y/L305V/V158D/E296V/K337A/S314E-FVII, F374Y/L305V/V158D/M298Q/K337A/S314E-FVII, F374Y/L305V/E296V/M298Q/K337A/V158T/S314E-FVII, F374Y/L305V/V158D/E296V/M298Q/K337A/S314E-FVII, S52A-Factor VII, S60A-Factor VII; R152E-Factor VII, S344A-Factor VII, T106N-FVII, K143N/N145T-FVII, V253N-FVII, R290N/A292T-FVII, G291N-FVII, R315N/V317T-FVII, K143N/N145T/R315N/V317T-FVII; and FVII having substitutions, additions or deletions in the amino acid sequence from 233Thr to 240Asn; FVII having substitutions, additions or deletions in the amino acid sequence from 304Arg to 329Cys; FVII having substitutions, additions or deletions in the amino acid sequence from 153Ile to 223Arg, and 406C-FVII.

Thus, substitution variants in a factor VII polypeptide include, without limitation substitutions in positions P10, K32, L305, M306, D309, L305, L305, F374, V158, M298, V158, E296, K337, M298, M298, S336, S314, K316, K316, F374, S52, S60, R152, S344, T106, K143, N145, V253, R290, A292, G291, R315, V317, and substitutions, additions or deletions in the amino acid sequence from T233 to N240 or from R304 to C329; or from I153 to R223, or combinations thereof, in particular variants such as P10Q, K32E, L305V, M306D, D309S, . L305I, L305T, F374P, V158T, M298Q, V158D, E296V, K337A, M298Q, M298K, S336G, S314E, K316H, K316Q, F374Y, S52A, S60A, R152E, S344A, T106N, K143N, N145T, V253N, R290N, A292T, G291N, R315N, V317T, and substitutions, additions or deletions in the amino acid sequence from T233 to N240, or from R304 to C329, or from I153 to R223, or combinations thereof.

The biological activity of Factor VIIa in blood clotting derives from its ability to (i) bind to Tissue Factor (TF) and (ii) catalyze the proteolytic cleavage of Factor IX or Factor X to produce activated Factor IX or X (Factor IXa or Xa, respectively).

For the purposes of the invention, biological activity of Factor VII polypeptides ("Factor VII biological activity") may be quantified by measuring the ability of a preparation to promote blood clotting, cf. Assay 4 described herein. In this assay, biological activity is expressed as the reduction in clotting time relative to a control sample and is converted to "Factor VII units" by comparison with a pooled human serum standard containing 1 unit/mL Factor VII activity. Alternatively, Factor VIIa biological activity may be quantified by (i) measuring the ability of Factor VIIa or a Factor VII-related polypeptide to produce activated Factor X (Factor Xa) in a system comprising TF embedded in a lipid membrane and Factor X. (Persson et al., J. Biol. Chem. 272:19919-19924, 1997); (ii) measuring Factor X hydrolysis in an aqueous system ("In Vitro Proteolysis Assay", see Assay 2 below); (iii) measuring the physical binding of Factor VIIa or a Factor VII-related polypeptide to TF using an instrument based on surface plasmon resonance (Persson, FEBS Letts. 413: 359-363, 1997); (iv) measuring hydrolysis of a synthetic substrate by Factor VIIa and/or a Factor VII-related polypeptide ("In Vitro Hydrolysis Assay", see Assay 1 below); or (v) measuring generation of thrombin in a TF-independent in vitro system (see Assay 3 below).

Factor VII variants having substantially the same or improved biological activity relative to wild-type Factor VIIa encompass those that exhibit at least about 10%, preferably at least about 25%, more preferably at least about 50%, more preferably at least about 75%, and most preferably at least about 90% of the specific activity of Factor VIIa that has been produced in the same cell type, when tested in one or more of a clotting assay (Assay 4), proteolysis assay (Assay 2), or TF binding assay as described above. Factor VII variants having substantially reduced biological activity relative to wild-type Factor VIIa are those that exhibit less than about 25%, preferably less than about 10%, more preferably less than about 5% and most preferably less than about 1% of the specific activity of wild-type Factor VIIa that has been produced in the same cell type when tested in one or more of a clotting assay (Assay 4), proteolysis assay (Assay 2), or TF binding assay as described above. Factor VII variants having a substantially modified biological activity relative to wild-type Factor VII include, without limitation, Factor VII variants that exhibit TF-independent Factor X proteolytic activity and those that bind TF but do not cleave Factor X.

Variants of Factor VII, whether exhibiting substantially the same or better bioactivity than wild-type Factor VII, or, alternatively, exhibiting substantially modified or reduced bioactivity relative to wild-type Factor VII, include, without limitation, polypeptides having an amino acid sequence that differs from the sequence of wild-type Factor VII by insertion, deletion, or substitution of one or more amino acids.

The expression "polypeptides" in connection with the terms "Factor X polypeptides" and "Factor IX polypeptides" is intended to encompass any protein comprising the amino acid sequence of the wild-type human Factor X and Factor IX, respectively, as well as the respective "variants", "related polypeptides", "derivatives" and "conjugates" thereof, where the expressions "variants", "related polypeptides", "derivatives" and "conjugates" are defined as for Factor VII, *mutatis mutandis.*

In some embodiments, the vitamin K-dependent polypeptide is a Factor VII polypeptide.

In some embodiments, the Factor VII polypeptide is human Factor VIIa (hFVIIa), preferably recombinantly made human Factor VIIa (rhVIIa).

In other embodiments, the Factor VII polypeptide is a Factor VII sequence variant.

In some embodiments, the Factor VII polypeptide has a glycosylation different from wild-type human Factor VII.

In some embodiments, the Factor VII polypeptide has substantially the same or increased half life in blood plasma compared to recombinant wild type human Factor VIIa.

In the currently most interesting embodiment, the protein is a Factor VII polypeptide, preferably a Factor VII polypeptide in activated form.

In the initial liquid pharmaceutical preparation, it is often desirable that the concentration of the active ingredient is such that the application of a unit dose does not cause unnecessary discomfort to the patient. Thus, a unit dose of more than about 2-10 mL is often undesirable. For the purpose of the present invention, the concentration of the protein (e.g. a Factor VII polypeptide) is therefore typically at least 0.01 mg/mL. In different embodiments, the protein (e.g. Factor VII polypeptide) is present in a concentration of 0.01-40 mg/mL; 0.01-20 mg/mL; 0.1-10 mg/mL; 0.5-5.0 mg/mL; 0.6-4.0 mg/mL; 1.0-4.0 mg/mL; 0.1-5 mg/mL; 0.1-4.0 mg/mL; 0.1-2 mg/mL; or 0.1-1.5 mg/mL.

A Factor VIIa concentration is conveniently expressed as mg/mL or as IU/mL, with 1 mg usually representing 43,000 - 56,000 IU or more.

Non-metallic inhibitor

The non-metallic inhibitor for a protein of the before-mentioned type is - of course - selected with due respect to the required stability of the protein in the initial liquid pharmaceutical preparation.

The term "inhibitor" is intended to encompass any compound with is capable of stabilising the initial liquid pharmaceutical preparation by suppressing or eliminating the auto-catalytic degradation of the protein in question.

The initial liquid pharmaceutical preparation should typically be stable for at least six months, and preferably up to 36 months, when stored at temperatures ranging from 2°C to 8°C. It should be understood that the initial liquid pharmaceutical preparation preferably is stable even at higher temperatures, such as ambient temperature, e.g. 20°C to 30°C, although this often requires a higher content of the inhibitor.

The term "stable" is intended to denote that after storage for 6 months at 2°C to 8°C the initial liquid pharmaceutical preparation retains at least 50% of its initial biological activity. Preferably, the initial liquid pharmaceutical preparation retains at least 70%, such as at least 80%, or at least 85%, or at least 90%, or at least 95%, of its initial activity after storage for 6 months at 2 to 8°C.

With respect to Factor VII polypeptides, the term "stable" is more particularly intended to denote that (i) after storage for 6 months at 2°C to 8°C the initial liquid pharmaceutical preparation retains at least 50% of its initial biological activity as measured by a one-stage clot assay (Assay 4), <u>or</u> (ii) after storage for 6 months at 2°C to 8°C, the content of heavy chain degradation products is at the most 40% (w/w) assuming that the initial liquid pharmaceutical preparation comprises no heavy chain degradation products (i.e. only the Factor VII polypeptide is entered into the calculation of the percentage). Preferably, the initial liquid pharmaceutical preparation retains at least 70%, such as at least 80%, or at least 85%, or at least 90%, or at least 95%, of its initial activity after storage for 6 months at 2 to 8°C. Also preferably, the content of heavy chain degradation products in the initial liquid pharmaceutical preparation is at the most 30% (w/w), at the most 25% (w/w), at the most 20% (w/w), at the most 15% (w/w), at the most 10% (w/w), at the most 5% (w/w), or at the most 3% (w/w).

Typically, the inhibitor is a non-metallic compound having a molecular weight of at the most 1000 Da, e.g. at the most 750 Da, such as at the most 500 Da.

The inhibitor is preferably a competitive inhibitor.

Examples of particularly useful inhibitors, in particular for Factor VII polypeptides, are those selected from the group consisting of amidines and guanidines, e.g. those selected from the group consisting of benzamidines and guanidines, more particular those disclosed in WO 2005/016365 A.

Thus, in one embodiment, the protein is a Factor VII polypeptide and the inhibitor is selected from the group consisting of benzamidines and guanidines.

Hence, one group of particularly interesting inhibitors (commonly referred to as stabilising agents (ii)) are those comprising a $-C(=N-Z^1-R^1)-NH-Z^2-R^2$ motif, wherein $Z^1$ and $Z^2$ independently are selected from the group consisting of -O-, -S-, $-NR^H-$ and a single bond, where $R^H$ is selected from the group consisting of hydrogen, $C_{1-4}$-alkyl, aryl and arylmethyl, and $R^1$ and $R^2$ independently are selected from the group consisting of hydrogen, optionally substituted $C_{1-6}$-alkyl, optionally substituted $C_{2-6}$-alkenyl, optionally substituted aryl, optionally substituted heterocyclyl, or

$Z^2$ and $R^2$ are as defined above and $-C=N-Z^1-R^1$ forms part of a heterocyclic ring, or

$Z^1$ and $R^1$ are as defined above and $-C-NH-Z^2-R^2$ forms part of a heterocyclic ring, or

$-C(=N-Z^1-R^1)-NH-Z^2-R^2$ forms a hetercyclic ring wherein $-Z^1-R^1-R^2-Z^2-$ is a biradical.

The term "$C_{1-6}$-alkyl" is intended to encompass acyclic and cyclic saturated hydrocarbon residues which have 1-6 carbon atoms and which can be linear or branched. Particular examples are methyl, ethyl, n-propyl, isopropyl, cyclopropyl, n-butyl, isobutyl, sec-butyl, tert-butyl, cyclopropylmethyl, n-pentyl, isopentyl, n-hexyl, etc. Similarly, the term "$C_{1-4}$-alkyl" encompasses acyclic and cyclic saturated hydrocarbon residues which have 1-4 carbon atoms and which can be linear or branched.

Similarly, the term "$C_{2-6}$-alkenyl" is intended to encompass acyclic and cyclic hydrocarbon residues which have 2-6 carbon atoms and comprise one unsaturated bond, which can be linear or branched. Examples of $C_{2-6}$-alkenyl groups are vinyl, allyl, but-1-en-1-yl, but-2-en-1-yl, pent-1-en-1-yl, and hex-1-en-1-yl.

The term "optionally substituted" in connection with $C_{1-6}$-alkyl and $C_{2-6}$-alkenyl groups is intended to denote that the group in question may be substituted one or several times, preferably 1-3 times, with group(s) selected from the group consisting of hydroxy, $C_{1-6}$-alkoxy (i.e. $C_{1-6}$-alkyl-oxy), $C_{2-6}$-alkenyloxy, oxo (forming a keto or aldehyde functionality), aryl, aryloxy, arylcarbonyl, heterocyclyl, heterocyclyloxy, heterocyclylcarbonyl, amino, mono- and di ($C_{1-6}$-alkyl)amino, halogen; where any aryl and heterocyclyl may be substituted as specifically described below for optionally substituted aryl and heterocyclyl.

"Halogen" includes fluoro, chloro, bromo, and iodo.

When used herein, the term "aryl" is intended to denote a fully or partially aromatic carbocyclic ring or ring system, such as phenyl, naphthyl, 1,2,3,4-tetrahydronaphthyl, anthracyl, phenanthracyl, pyrenyl, benzopyrenyl, fluorenyl and xanthenyl, among which phenyl is a preferred example.

The term "heterocyclyl" is intended to denote a saturated, partially unsaturated, partially aromatic or fully aromatic carbocyclic ring or ring system where one or more of the carbon atoms have been replaced with heteroatoms, e.g. nitrogen (=N- or -NH), sulphur (-S-), and/or oxygen (-O-) atoms. Examples of such heterocyclyl groups are oxazolyl, oxazolinyl, oxazolidinyl, isoxazolyl, isoxazolinyl, isoxazolidinyl, oxadiazolyl, oxadiazolinyl, oxadiazolidinyl, thiazolyl, isothiazolyl, pyrrolyl, pyrrolinyl, pyrrolidinyl, imidazolyl, imidazolinyl, imidazolidinyl, pyrazolyl, pyridinyl, pyrazinyl, pyridazinyl, piperidinyl, coumaryl, furyl, quinolyl, benzothiazolyl, benzotriazolyl, benzodiazolyl, benzoxozolyl, diazolyl, diazolinyl, diazolidinyl, triazolyl, triazolinyl, triazolidinyl, tetrazol, etc. Preferred heterocyclyl groups are 5-, 6- or 7-membered monocyclic groups such as isoxazolyl, isoxazolinyl, oxadiazolyl, oxadiazolinyl, pyrrolyl, pyrrolinyl; diazolyl, diazolinyl, triazolyl, triazolinyl, imidazolyl, imidazolinyl, etc.

The term "heterocyclic ring" is intended to mean a ring corresponding to those defined under "heterocyclyl".

In connection with the terms "aryl", "heterocyclyl" and "heterocyclic ring", the term "optionally substituted" is intended to denote that the group in question may be substituted one or several times, preferably 1-3 times, with group (s) selected from hydroxy (which when present in an enol system may be represented in the tautomeric keto form), $C_{1-6}$-alkyl, $C_{2-6}$-alkenyl, phenyl, benzyl, $C_{1-6}$-alkoxy, oxo (which may be represented in the tautomeric enol form), carboxy, $C_{1-6}$-alkoxycarbonyl, $C_{1-6}$-alkylcarbonyl, amino, mono- and di($C_{1-6}$-alkyl)amino, dihalogen-$C_{1-4}$-alkyl, trihalogen-$C_{1-4}$-alkyl, and halogen. The most typical examples of substituents are hydroxyl, $C_{1-4}$-alkyl, phenyl, benzyl, $C_{1-4}$-alkoxy, oxo, amino, mono- and dimethylamino and halogen.

Besides the fact that $R^1$ and $R^2$ independently can be selected from the group consisting of hydrogen, optionally substituted $C_{1-6}$-alkyl, optionally substituted $C_{2-6}$-alkenyl, optionally substituted aryl, optionally substituted heterocyclyl, it is also possible that a part of the -C(=N-$Z^1$-$R^1$)-NH-$Z^2$-$R^2$ motif may be part of a hetercyclic ring, while the other part of the motif has the meaning defined for $Z^1$, $Z^2$, $R^1$ and $R^2$, respectively. In some interesting embodiments, -C=N-$Z^1$-$R^1$ may form part of a heterocyclic ring selected from the group consisting of a 1,2-diazole ring, an isoxazole ring, a 1,2,4-triazole ring, and a 1,2,4-oxadiazole ring, or -C-NH-$Z^2$-$R^2$ may form part of a heterocyclic ring selected from the group consisting of a 1,2-diazoline ring, an isoxazoline ring, a 1,2,4-triazoline ring, and a 1,2,4-oxadiazoline ring. Such heterocyclic rings may be substituted as described above.

In some embodiments, at least one of $R^1$ and $R^2$ is hydrogen, e.g. both are hydrogen. Further, in some embodiment, which may be combined with the embodiments mentioned before, at least one of $Z^1$ and $Z^2$ is a single bond, e.g. both are a single bond. In special embodiments, $R^1$ and $R^2$ are both hydrogen, and $Z^1$ and $Z^2$ are both a single bond.

It is believed that the -C(=N-$Z^1$-$R^1$)-NH-$Z^2$-$R^2$ motif is particularly important for the stabilising effect of the stabilising agent (ii). In particular, it is believed that the -C(=N-$Z^1$-$R^1$)-NH-$Z^2$-$R^2$ motif mimics an arginine moiety of a substrate for the Factor VII polypeptide.

In more specific embodiments, the stabilising agent (ii) is at least one selected from the group consisting of amidine compounds comprising a -C-C(=N-$Z^1$-$R^1$)-NH-$Z^2$-$R^2$ motif and guanidines compounds comprising a >N-C(=N-$Z^1$-$R^1$)-NH-$Z^2$-$R^2$ motif.

In some embodiments, the stabilising agent (ii) is at least one amidine compound selected from the group consisting of benzamidines comprising the motif -$C_6H_4$-C(=N-$Z^1$-$R^1$)-NH-$Z^2$-$R^2$, wherein $C_6H_4$ denotes an optionally substituted benzene ring, of which benzamidine ($R^1$ and $R^2$ are hydrogen and $Z^1$ and $Z^2$ are a single bond) constitutes a particular embodiment (see the Experimental section).

In other particular embodiments thereof, the benzamidines comprises the motif >N-$C_6H_4$-C(=N-$Z^1$-$R^1$)-NH-$Z^2$-$R^2$, wherein $C_6H_4$ denotes an optionally substituted benzene ring, i.e. an o-amino-benzamidine, a m-amino-benzamidine or a p-amino-benzamidine, of which p-amino-benzamidines, such as p-amino-benzamidine, are the currently most promising.

Further illustrative examples of p-amino-benzamidines are those disclosed by Aventis in EP 1 162 194 A1, cf. in particular those defined in claims 1-6 and in sections [0009]-[0052], and in EP 1 270 551 A1, cf. in particular claims 1 and 2 and sections [0010]-[0032].

In another embodiment, the stabilising agent (ii) is at least, one guanidine compound selected from the group consisting of guanidines compounds comprising a -$CH_2$-NH-C(=N-$Z^1$-$R^1$)-NH-$Z^2$-$R^2$ motif. Examples of guanidine compounds are those selected from the group consisting of arginine, arginine derivatives and peptides of 2-5 amino acid residues comprising at least one arginine residue. Arginine constitutes a particular embodiment (see the Experimental section).

The term "arginine derivatives" is intended to encompass arginine homologues, N-terminal functionalised arginines (e.g. N-methylated and N-acylated (e.g. acetylated) derivatives), C-terminal functionalised arginines (e.g. C-amidated, C-alkylamidated, and C-alkylated derivatives), and combinations thereof.

As mentioned above, the one crucial motif of the stabilising agents is -C(=N-$Z^1$-$R^1$)-NH-$Z^2$-$R^2$. Other parts of the stabilising agent may also be important, in particular with respect to optimisation of the stabilising effect and the tolerance by the patient. Typically, the stabilising agent has the formula Y-C(=N-$Z^1$-$R^1$)-NH-$Z^2$-$R^2$, wherein Y is an organic radical. The radical Y is typically selected in order to improve the efficiency of the stabilising effect. Also, the radical Y may comprise one or more further motifs of the formula -C(=N-$Z^1$-$R^1$)-NH-$Z^2$-$R^2$.

The concentration of the stabilising agent (or agents) (ii) is typically at least 1 μM. The desirable (or necessary) concentration typically depends on the selected stabilising agent (or agents), more specifically on the binding affinity of the selected stabilising agent to the Factor VII polypeptide.

In different embodiments, the stabilising agent (ii) is present in a concentration of at least 5 μM, at least 10 μM, at least 20 μM, at least 50 μM, at least 100 μM, at least 150 μM, at least 250 μM, at least 500 μM, at least 1 mM, at least 2 mM, at least 4 mM, at least 5 mM, at least 8 mM, at least 9 mM, at least 10 mM, at least 15 mM, at least 20 mM, such as 20-2000 μM, 50-5000 μM, 0.1-10 mM, 0.2-20 mM, or 0.5-50 mM.

In one embodiment, the stabilising agent (ii) is benzamidine and the concentration of said agent is at least 2 mM, although it is envisaged that substituted benzamidines may be more potent for what reason they can be added in lower concentrations.

9

In one embodiment, the stabilising agent (ii) is arginine and the concentration of said agent is at least 10 mM.

Other constituents

In order to render the resulting liquid pharmaceutical preparation useful for direct parenteral administration to a mammal such as a human, it is normally required that the pH value of the initial liquid pharmaceutical preparation (and thereby also the resulting liquid pharmaceutical preparation) is held within reasonable limits, such as from about 4.0 to about 9.0, such as about 5.0 to about 9.0. To ensure a suitable pH value under the conditions given, the liquid pharmaceutical preparation also comprises a buffering agent (iii) suitable for keeping pH in the range of from about 4.0 to about 9.0, such as about 5.0 to about 9.0.

The term "buffering agent" encompasses those agents or combinations of agents which maintain the solution pH in an acceptable range from about 5.0 to about 9.0.

In one embodiment, the buffering agent (iii) is at least one component selected from the groups consisting of acids and salts of MES, PIPES, ACES, BES, TES, HEPES, TRIS, histidine, imidazole, glycine, glycylglycine, glycinamide, phosphoric acid, acetic acid (e.g. sodium or calcium acetate), lactic acid, glutaric acid, citric acid, tartaric acid, malic acid, maleic acid, and succinic acid. It should be understood that the buffering agent may comprise a mixture of two or more components, wherein the mixture is able to provide a pH value in the specified range. As examples can be mentioned acetic acid and sodium acetate, etc.

The concentration of the buffering agent is chosen so as to maintain the preferred pH of the solution. In various embodiments, the concentration of the buffering agent is 1-100 mM; 1-50 mM; 1-25 mM; or 2-20 mM.

In one embodiment, the pH of the composition is kept from about 5.0 to about 8.0; such as from about 5.0 to about 7.5; from about 5.0 and about 7.0; from about 5.0 to about 6.5, from about 5.0 to about 6.0, from about 5.5 to about 7.0; from about 5.5 to about 6.5, from about 6.0 to about 7.0, from about 6.4 to about 6.6, or from about 5.2 to about 5.7.

In addition to the protein, the inhibitor and the buffering agent, the initial liquid pharmaceutical preparation may comprise additional components beneficial for the preparation, formulation, stability, or administration of the composition.

Hence, the initial liquid pharmaceutical preparation may also include non-ionic surfactant(s), tonicity modifying agent(s) (including ionic strength modifying agent(s)), antioxidants, preservatives, etc., e.g. as disclosed in WO 2005/016365 A.

Sealed container

Due to the fact that the initial liquid pharmaceutical preparation may be prepared several months before the actual use thereof, it is necessary to provide the preparation in a sealed environment so as to facilitate storage. Hence, the initial liquid pharmaceutical preparation is provided in a sealed container, e.g. a vial, cartridge, ampoule, carpoule, etc. which, on the one hand, ensures that the integrity of the preparation is preserved and; on the other hand, allows for easy access to the preparation whenever the preparation is to be prepared for administration as defined in steps (b) and (c).

Thus, the initial liquid pharmaceutical preparation is typically provided in an air-tight container (e.g. a vial or a cartridge (such as a cartridge for a pen applicator)), optionally including an inert gas to occupy any void space in the container. The inert gas may be selected from the groups consisting of nitrogen, argon, etc. The container (e.g. vial or cartridge) is typically made of glass or plastic, in particular glass, optionally closed by a rubber septum or other closure means allowing for penetration with preservation of the integrity of the pharmaceutical composition. In a further embodiment, the container is a vial or cartridge enclosed in a sealed bag, e.g. a sealed plastic bag, such as a laminated (e.g. metal (such as aluminium) laminated plastic bag).

In various embodiments, the amount of the pharmaceutical preparation in said sealed container corresponds to 1-10 doses, e.g. to 1-5 doses, in particular to 1 dose.

*Method step (b)*

In a subsequent step (b), the pharmaceutical preparation is contacted with a solid phase polymeric material capable of retaining at least a substantial portion of said inhibitor.

An important feature of the contacting step is that a substantial portion of the inhibitor is bound to the solid phase polymeric material whereas the pharmaceutically active protein is substantially unbound and preserves its biological activity.

Solid phase polymeric material

The solid phase polymeric material may be selected from a fairly broad range of materials, including, but not limited to, materials known as solid phase materials used for separation purposes, e.g. in chromatographic methods.

Suitable examples of types of materials are those selected from the group consisting of cation-exchange materials, anion exchange materials, polar materials, non-polar materials, hydrophilic materials, hydrophobic materials, and gel-filtration materials, of which ionexchange materials, in particular cationic exchange resins, are currently preferred.

One particular feature of preferred polymeric materials is that the activity of the protein belonging to the group of serine proteases/Vitamin K-dependent proteins remains substantially unchanged (i.e. at least 90% of the initial activity is preserved) upon contact with the polymeric material for 10 minutes.

Currently most interesting examples of materials are those selected from cellulose fibres, and hydrophilic synthetic polymers such as methacrylates and styrene copolymers (e.g. polystyrene/divinylbenzene (PSDVB)).

Currently most interesting commercial products are Chelex 100 (BioRad Laboratories) which is a cation-exchange material.

The solid phase polymeric material must be capable of retaining at least a substantial portion of the inhibitor, while leaving a substantial portion of the protein unbound. By the term "substantially" is typically meant "at least 75%", but preferably at least 80%, such as at least 90% or even at least 95%.

For a given inhibitor, suitable solid phase polymeric materials that will retain at least a substantial portion of the inhibitor can be predicted by using Hansen Solubility parameters (HSP) (see: Charles M. Hansen, Hansen's Solubility Parameters: A User's Handbook, CRC Press, 1999).

By investigating solubility parameters (HSP), compatibility, adsorption on surfaces, orientation toward materials of similar affinities (self-assembly), and other phenomena associated with solubility and affinity can be predicted. Liquids with similar solubility parameter will be miscible, and polymers will dissolve or adsorb liquids whose solubility parameter are not to different from their own. The basic principle has been "like dissolve like" and more recently this has been modified to "like seeks like". Solubility parameters (HSP) help put numbers into this simple qualitative idea and have been extensively used and proven valuable to a variety of industries including the biological field.

In the present invention, solubility parameters (HSP) of the inhibitor(s) of interest are compared to the solubility parameters (HSP) of the solid phase polymeric materials of interest. Suitable polymeric materials for binding said inhibitor(s) are then selected based on similarity of their solubility parameters ("like seeks like").

For example, calculations made on the basis of benzamidine shows that affinity is expected within cellulose derivates, polymers based on acrylate, acrylate co-polymers, vinyl acetate (PVA), vinyl butyral (PVB), acrylonitril, styrene co-polymers, vinylpyrrolidone (PVP) and as well as polymers holding an acid group.

Soluble polymer like PVP and PVA can be cross linked for stabilising the physical form.

The bulk of the polymeric materials may be arranged in various ways, e.g. in the form of a filter, such in the form of a plug filter or a disc filter, a column or the bulk may be coated on the inner surface of a tube, needle or chamber.

Depending of the form of the bulk of the polymeric materials, the initial liquid pharmaceutical preparation may be contacted therewith in a batch-wise manner or by passing through or over the bulk.

In one currently preferred embodiment, the liquid pharmaceutical preparation is contacted with the polymeric material by passing the liquid pharmaceutical preparation through a bulk of the polymeric material. In this embodiment, the bulk is preferably in the form of a filter, such in the form of a plug filter or a disc filter, or a column.

In another embodiment, the liquid pharmaceutical preparation is contacted with the polymeric material by passing the liquid pharmaceutical preparation over a bulk of the polymeric material. In this embodiment, the bulk is preferably coated on the inner surface of a tube, needle or chamber.

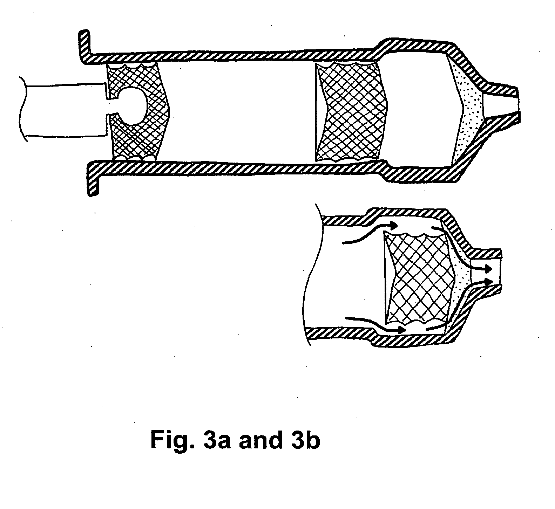

In order to avoid contamination and easy removal of the inhibitor substance, the liquid formulation may, for example, be contained in a syringe or other compartment and in the outlet zone the polymeric material are situated (see Figure 3a and 3b). During injection the liquid formulation passes through the polymeric solid material and the inhibitor are removed.

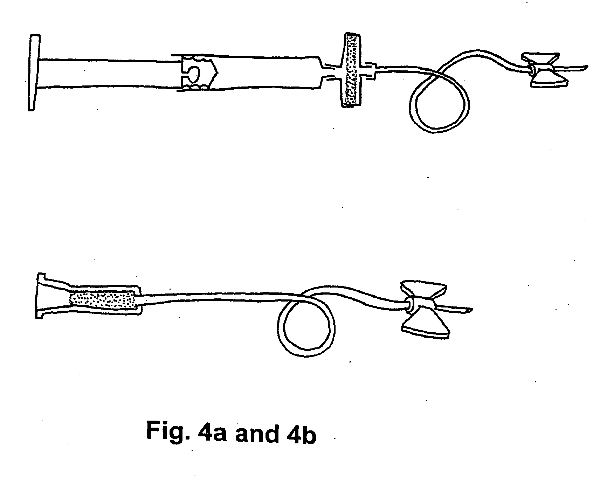

In another embodiment, the liquid formulation is contained in a syringe or other compartment. Just before injection a sterile filter holding the polymeric material are connected with the intravenous injection tube where a needle, e.g. a butterfly needle, is mounted on the outlet. During injection the inhibitor are removed (see Figure 4a and 4b showing two alternative examples of said sterile filters).

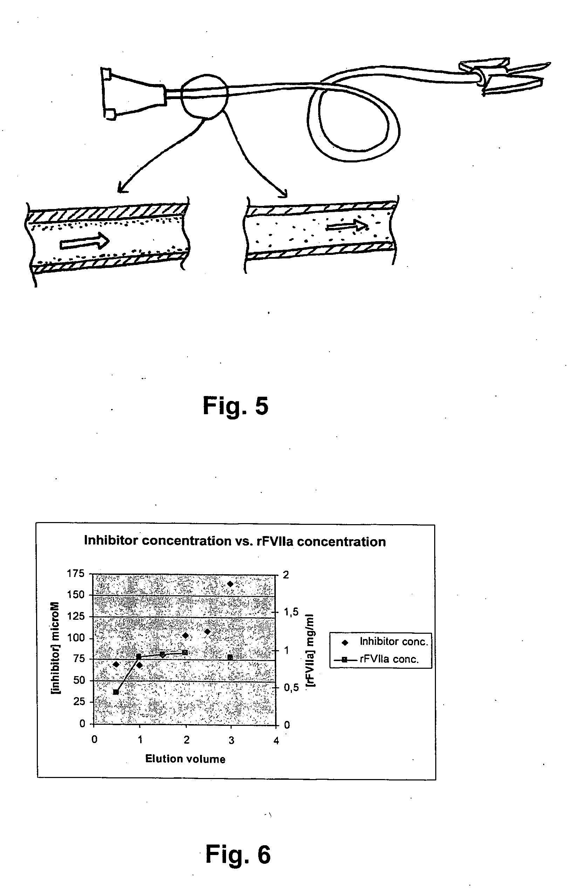

In another embodiment, the liquid formulation is contained in a syringe or other compartment. Just before injection an injection tube with a needle, e.g. a butterfly needle, is mounted on the outlet. The tube holds the polymeric material either coated on the inside or filled in the tube. During injection the inhibitor are removed (see Figure 5).

*Method step (c)*

In a subsequent step (c), the liquid pharmaceutical preparation is separated from said solid phase polymeric material so as to obtain a resulting liquid pharmaceutical preparation having a reduced concentration of said inhibitor compared to the initial liquid pharmaceutical preparation.

Depending on the actual performance of step (b), the resulting liquid pharmaceutical preparation may already be obtained in a form separated from the solid phase polymeric materials, in particular if the initial liquid pharmaceutical preparation is passed through or over a bulk of the solid phase polymeric material. However, if not already obtained in separated form, the resulting liquid pharmaceutical preparation must be separated from the solid phase polymeric material, e.g. by filtration upon application of a moderate pressure, e.g. by means of a plunger, by sedimentation, or the like.

Due to the binding thereof, the concentration of the non-metallic inhibitor is typically reduced by at least 75%, such as by at least 80%, or even at least 90% or at least 95%, or virtually eliminated. Thus, the concentration of the inhibitor can be brought to a level where otherwise detrimental therapeutical effects can be eliminated.

In one currently preferred embodiment, the amount of the pharmaceutical preparation in the sealed container corresponds to 1 dose, and the dose is passed through or is passed over a bulk of the polymeric material.

In another preferred embodiment, the amount of the pharmaceutical preparation in said sealed container corresponds to 2-10 doses, and an amount corresponding to one dose is passed through or is passed over a bulk of the polymeric material.

Typically, the contact time in step (b) is in the range of 0.1-100 sec, typically 0.5-30 sec.

*Method of use*

In one variant, the method of the present invention is followed by the step of administering a therapeutically or prophylactically effective amount of the resulting liquid pharmaceutical preparation to a subject, in particular to a human.

Typically, this step is conducted within 60 minutes from the completion of step (c), in particular within 5 minutes from the completion of step (c), and preferably immediately after completion of step (c), or possibly in conjunction with step (c).

The preparations may be used to treat any Factor VII-responsive syndrome, such as, e.g., bleeding disorders, including those caused by clotting Factor deficiencies (e.g., e.g. haemophilia A, haemophilia B, coagulation Factor XI deficiency, coagulation Factor VII deficiency); by thrombocytopenia or von Willebrand's disease, or by clotting Factor inhibitors, and intra cerebral haemorrhage, or excessive bleeding from any cause. The preparations may also be administered to patients in association with surgery or other trauma or to patients receiving anticoagulant therapy.

The term "effective amount" is the effective dose to be determined by a qualified practitioner, who may titrate dosages to achieve the desired response. Factors for consideration of dose will include potency, bioavailability, desired pharmacokinetic/pharmacodynamic profiles, condition of treatment, patient-related factors (e.g. weight, health, age, etc.), presence of co-administered medications (e.g., anticoagulants), time of administration, or other factors known to a medical practitioner.

The term "treatment" is defined as the management and care of a subject, e.g. a mammal, in particular a human, for the purpose of combating the disease, condition, or disorder and includes the administration of a protein to prevent the onset of the symptoms or complications, or alleviating the symptoms or complications, or eliminating the disease, condition, or disorder. The pharmaceutical compositions containing a therapeutically active protein may be administered parenterally to subjects in need of such a treatment. Parenteral administration may be performed by subcutaneous, intramuscular or intravenous injection by means of a syringe, optionally a pen-like syringe. Alternatively, parenteral administration can be performed by means of an infusion pump.

The pharmaceutical composition is preferably adapted to subcutaneous, intramuscular or intravenous injection according to methods known in the art.

Typically, the resulting liquid pharmaceutical preparation is administered by injection as described above.

Also as mentioned above, a subject in need of the therapeutically active protein is typically suffering from, or is in risk of gaining, a Factor VII-responsive syndrome.

*Preferred embodiments*

A currently preferred embodiment relates to a method of removing a protein inhibitor from a liquid pharmaceutical preparation, said method comprising the steps of:

(a) providing a sealed container comprising an initial liquid pharmaceutical preparation comprising a Factor VII polypeptide and an inhibitor selected from benzamidines and guanidines;

(b) contacting said liquid pharmaceutical preparation with a solid phase polymeric material, in particular a cation-exchange material, capable of retaining at least a substantial portion of said inhibitor, said polymeric materials being selected from the group consisting of cellulose fibres and hydrophilic synthetic polymers; and

(c) separating said liquid pharmaceutical preparation from said solid phase polymeric material so as to obtain a resulting liquid pharmaceutical preparation having a reduced concentration of said inhibitor compared to the initial liquid pharmaceutical preparation.

A list of embodiments of the present invention is given below:

Embodiment 1. A method of removing a protein inhibitor from a liquid pharmaceutical preparation, said method comprising the steps of:

(a) providing a sealed container comprising an initial liquid pharmaceutical preparation comprising (i) a protein belonging to the group of serine proteases/Vitamin K-dependent proteins and (ii) a non-metallic inhibitor for said protein;

(b) contacting said pharmaceutical preparation with a solid phase polymeric material capable of retaining at least a substantial portion of said inhibitor; and

(c) separating said liquid pharmaceutical preparation from said solid phase polymeric material so as to obtain a resulting liquid pharmaceutical preparation having a reduced concentration of said inhibitor compared to the initial liquid pharmaceutical preparation.

Embodiment 2. The method according to embodiment 1, wherein the protein has autocatalytic properties.

Embodiment 3. The method according to any one of the preceding embodiments, wherein the protein is in partly or fully activated form.

Embodiment 4. The method according to any one of the preceding embodiments, wherein the protein is selected from the group consisting of GAS-6, Protein S, Factor II (Prothrombin), Factor X polypeptides, Factor IX polypeptides, Protein C, Factor VII polypeptides, Protein Z, Transmembrane gamma-carboxyglutamic acid protein 1, Transmembrane gamma-carboxyglutamic acid protein 2, Transmembrane gamma carboxyglutamic acid protein 3, Transmembrane gamma-carboxyglutamic acid protein 4, Matrix Gla protein, and Osteocalcin.

Embodiment 5. The method according to any one of the preceding embodiments, wherein the protein is a Vitamin K-dependent coagulation factor selected from the group consisting of Factor VII polypeptides, Factor IX polypeptides, Factor X polypeptides and activated Protein C.

Embodiment 6. The method according to any one of the preceding embodiments, wherein the protein is a Factor VII polypeptide, preferably a Factor VII polypeptide in activated form.

Embodiment 7. The method according to any one of the preceding embodiments, wherein the inhibitor is a non-metallic compound having a molecular weight of at the most 1000 Da, e.g. at the most 750 Da, such as at the most 500 Da.

Embodiment 9. The method according to any one of the preceding embodiments, wherein the inhibitor is selected from the group consisting of amidines and guanidines.

Embodiment 10. The method according to any one of the preceding embodiments, wherein the protein is a Factor VII polypeptide and the inhibitor is selected from the group consisting of benzamidines and guanidines.

Embodiment 11. The method according to any one of the preceding embodiments, wherein the polymeric material is selected from the group consisting of cation-exchange materials, anion exchange materials, polar materials, non-polar materials, hydrophilic materials, and hydrophobic materials.

Embodiment 12. The method according to any one of the preceding embodiments, wherein the polymeric material is selected from the group consisting of cellulose fibres and hydrophilic synthetic polymers.

Embodiment 13. The method according to any one of the preceding embodiment, wherein the polymeric material is selected from the group consisting of cation-exchange materials.

Embodiment 14. The method according to any one of the preceding embodiments, wherein the liquid pharmaceutical preparation is contacted with the polymeric material by passing the liquid pharmaceutical preparation through a bulk of the polymeric material.

Embodiment 15. The method according to any one of embodiments 1-13, wherein the liquid pharmaceutical preparation is contacted with the polymeric material by passing the liquid pharmaceutical preparation over a bulk of the polymeric material.

Embodiment 16. The method according to any one of the preceding embodiments, wherein the amount of the pharmaceutical preparation in said sealed container corresponds to 1-10 doses, e.g. 1-5 doses, in particular 1 dose.

Embodiment 17. The method according to embodiment 16, wherein the amount of the pharmaceutical preparation in said sealed container corresponds to 1 dose, and the dose is passed through or is passed over a bulk of the polymeric material.

Embodiment 18. The method according to embodiment 16, wherein the amount of the pharmaceutical preparation in said sealed container corresponds to 2-10 doses, and an amount corresponding to one dose is passed through or is passed over a bulk of the polymeric material.

Embodiment 19. The method according to any one of the preceding embodiments, wherein the contact time in step (b) is in the range of 0.1-100 sec.

Embodiment 20. A method according to any one of the preceding embodiments, said method comprising the steps of:

(a) providing a sealed container comprising an initial liquid pharmaceutical preparation comprising a Factor VII polypeptide and an inhibitor selected from benzamidines and guanidines;

(b) contacting said liquid pharmaceutical preparation with a solid phase polymeric material capable of retaining at least a substantial portion of said inhibitor, said polymeric materials being selected from the group consisting of cellulose fibres and hydrophilic synthetic polymers; and

(c) separating said liquid pharmaceutical preparation from said solid phase polymeric material so as to obtain a resulting liquid pharmaceutical preparation having a reduced concentration of said inhibitor compared to the initial liquid pharmaceutical preparation.

Embodiment 21. The method according to embodiment 20, wherein the polymeric material is selected from the group consisting of cation-exchange materials.

The present invention is illustrated by the followed non-limiting examples.

EXPERIMENTALS

*General methods*

Assays suitable for determining biological activity of Factor VII polypeptides

Factor VII polypeptides useful in accordance with the present invention may be selected by suitable assays that can be performed as simple preliminary in vitro tests. Thus, the present specification discloses a simple test (entitled "In Vitro Hydrolysis Assay") for the activity of Factor VII polypeptides.

1st generation clot assay

The activity of the Factor VII polypeptides may be measured using a one-stage clot assay essentially as described in WO 92/15686 or US 5,997,864. Briefly, the sample to be tested is diluted in 50 mM Tris (pH 7.5), 0.1% BSA and 100 $\mu$L is incubated with 100 $\mu$L of Factor VII deficient plasma and 200 $\mu$L of thromboplastin C containing 10 mM Ca2+. Clotting times are measured and compared to a standard curve using a reference standard or a pool of citrated normal human plasma in serial dilution.

<u>In Vitro Hydrolysis Assay (Assay 1)</u>

Native (wild-type) Factor VIIa and Factor VII polypeptide (both hereinafter referred to as "Factor VIIa") may be assayed for specific activities. They may also be assayed in parallel to directly compare their specific activities. The assay is carried out in a microtiter plate (MaxiSorp, Nunc, Denmark). The chromogenic substrate D-Ile-Pro-Arg-p-nitroanilide (S-2288, Chromogenix, Sweden), final concentration 1 mM, is added to Factor VIIa (final concentration 100 nM) in 50 mM HEPES, pH 7.4, containing 0.1 M NaCl, 5 mM $CaCl_2$ and 1 mg/mL bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used for calculating the ratio between the activities of Factor VII polypeptide and wild-type Factor VIIa:

$$Ratio = (A405\ nm\ Factor\ VII\ polypeptide)/(A405\ nm\ Factor\ VIIa\ wild\text{-}type).$$

Based thereon, Factor VII polypeptides with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

The activity of the Factor VII polypeptides may also be measured using a physiological substrate such as Factor X ("In Vitro Proteolysis Assay"), suitably at a concentration of 100-1000 nM, where the Factor Xa generated is measured after the addition of a suitable chromogenic substrate (eg. S-2765). In addition, the activity assay may be run at physiological temperature.

<u>In Vitro Proteolysis Assay (Assay 2)</u>

Native (wild-type) Factor VIIa and Factor VII polypeptide (both hereinafter referred to as "Factor VIIa") are assayed in parallel to directly compare their specific activities. The assay is carried out in a microtiter plate (MaxiSorp, Nunc, Denmark). Factor VIIa (10 nM) and Factor X (0.8 microM) in 100 μL 50 mM HEPES, pH 7.4, containing 0.1 M NaCl, 5 mM $CaCl_2$ and 1 mg/mL bovine serum albumin, are incubated for 15 min. Factor X cleavage is then stopped by the addition of 50 μL 50 mM HEPES, pH 7.4, containing 0.1 M NaCl, 20 mM EDTA and 1 mg/mL bovine serum albumin. The amount of Factor Xa generated is measured by the addition of the chromogenic substrate Z-D-Arg-Gly-Arg-p-nitroanilide (S-2765, Chromogenix, Sweden), final concentration 0.5 mM. The absorbance at 405 nm is measured continuously in a SpectraMax™ 340 plate reader (Molecular Devices, USA). The absorbance developed during 10 minutes, after subtraction of the absorbance in a blank well containing no FVIIa, is used for calculating the ratio between the proteolytic activities of Factor VII polypeptide and wild-type Factor VIIa:

$$Ratio = (A405\ nm\ Factor\ VII\ polypeptide)/(A405\ nm\ Factor\ VIIa\ wild\text{-}type).$$

Based thereon, a Factor VII polypeptide with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

<u>Thrombin generation assay (Assay 3)</u>

The ability of a Factor VII polypeptides to generate thrombin can be measured in an assay (Assay 3) comprising all relevant coagulation Factors and inhibitors at physiological concentrations (minus Factor VIII when mimicking hemophilia A conditions) and activated platelets (as described on p. 543 in Monroe et al. (1997) Brit. J. Haematol. 99, 542-547).

<u>One-stage Coagulation Assay (Clot Assay) (Assay 4)</u>

Factor VII polypeptides may also be assayed for specific activities ("clot activity") by using a one-stage coagulation assay (Assay 4). For this purpose, the sample to be tested is diluted in 50 mM PIPES-buffer (pH 7.2), 1% BSA and 40 μl is incubated with 40 μl of Factor VII deficient plasma and 80 μl of human recombinant tissue factor containing 10 mM $Ca^{2+}$ and synthetic phospholipids. Coagulation times (clotting, times) are measured and compared to a standard curve using a reference standard in a parallel line assay.

*Examples*

*Example 1*

The ability of Chelex 100 to remove inhibitor S-2-[3-(4-carbamimidoylphenyl)-ureido]-N-[1-(3-methoxyphenyl)-ethyl]-acetamide (inhibitor 0008) from an rFVIIa formulation was investigated in the following way:

A solution of rFVIIa bulk containing 1.4 mg/mL rFVIIa, 50 mM NaCl, 10 mM $CaCl_2$ $2H_2O$ and 10 mM Glycylglycine was thawn and mixed with a solution of 450 $\mu$M inhibitor 0008 in the ratio 2 parts of rFVIIa bulk and 1 part of inhibitor 0008 solution. 10 mM histidine was added to the solution and pH was adjusted to 6.50. The solution was divided into aliquots of 2.00 mL.

Chelex 100 was added in an increasing amount to the solution, the following amounts of Chelex was added: 0, 40, 80, 120, 160, 200, 240, 280 and 320 mg Chelex 100 per 2.00 mL of rFVIIa/inhibitor solution.

The solutions were stirred for 1 minute and filtered through a 0.22 $\mu$M filter. All solutions were analysed for content of inhibitor using High Performance Gel Permeation Chromatography (GP-HPLC).

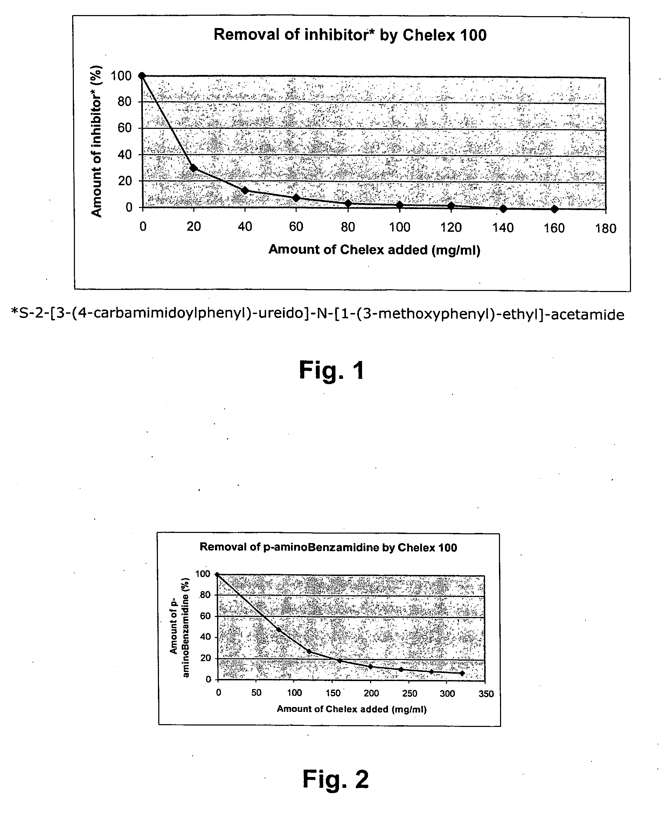

The GP-HPLC method was run on a Waters Protein Pak 300 SW column. 7.5x300 mm, using 0.2 M ammoniumsulfat pH 7.0 containing 5% isopropanol as the mobile phase. Flow rate: 0.5 mL/min and detection: 215 nm. The inhibitor appears in the chromatogram as a separate peak and the relative content can be calculated. The results are shown in Figure 1.

*Example 2*

The ability of Chelex 100 to remove p-aminoBenzamidine from a rFVIIa formulation was investigated in the following way:

A solution of rFVIIa bulk containing 1.4 mg/mL rFVIIa, 50 mM NaCl, 10 mM $CaCl_2$ $2H_2O$ and 10 mM Glycylglycine was thawn and mixed with a solution of 30 mM p-aminoBenzamidine in the ratio 2 parts of rFVIIa bulk and 1 part of p-aminoBenzamidine solution. 10 mM histidine was added to the solution and pH was adjusted to 6.50. The solution was divided into aliquots of 2.00 mL.

Chelex 100 was added in an increasing amount to the solution, the following amounts of Chelex was added: 0, 160, 240, 320, 400, 480, 560 and 640 mg Chelex 100 per 2.00 mL of rFVIIa/inhibitor solution.

The solutions were stirred for 1 minute and filtered through a 0.22 $\mu$M filter. All solutions were analysed for content of inhibitor using High Performance Gel Permeation Chromatography (GP-HPLC).

The GP-HPLC method was run on a Waters Protein Pak 300 SW column. 7.5x300 mm, using 0.2 M ammoniumsulfat pH 7.0 containing 5% isopropanol as the mobile phase. Flow rate: 0.5 mL/min and detection: 215 nm. The inhibitor appears in the chromatogram as a separate peak and the relative content can be calculated. The results are shown in Figure 2.

*Example 3*

The ability of Chelex 100 to remove benzamidine from a rFVIIa formulation is investigated in the following way:

5 ml of a 1mg/ml Factor VIIa solution stabilised by 50 mM benzamidine is added to 1 grams of Chelex 100 in a glass flask and is mixed by hand turning for 30 seconds.

Approximate 2 ml of the mixed solution is transferred to a centrifuge glass and then centrifuged for 5 minutes at 10.000 rotations per minute.

The supernatant holds a Factor VIIa solution where the content of benzamidine is reduced to a concentration of 10 mM.

*Example 4*

The ability of Chelex 100 to remove inhibitor 2-[3-(4-Carbamimidoylphenyl)-ureido]-N-[1-(3-methoxyphenyl)-ethyl]-acetamide from a rFVIIa formulation was investigated in the following way:

A (frozen) solution of rFVIIa bulk containing 1,4 mg/ml rFVIIa, 50 mM NaCl, 10 mM CaCl2 2H20 and 10 mM Glycylglycine was thawed and mixed with an inhibitor solution of 4,5 mM inhibitor 2-[3-(4-Carbamimidoylphenyl)-ureido]-N-[1-(3-methoxyphenyl)-ethyl]-acetamide containing 50 mM NaCl, 10 mM CaCl2 2H20 and 10 mM Glycylglycine.

The mixture ratio was: rFVIIa bulk : inhibitor solution = 2 : 1. the final solution 10 mM histidine was added and pH was adjusted to 6.50.

Chelex was prepared in the following way:

A NAP™ column from Pharmacia was emptied for column material which was replaced by Chelex 100 material corresponding to a column height of approximately 3 mm. The column was equilibrated with the following buffer: 50 mM NaCl, 10 mM Histidine and 10 mM Glycylglycine, pH = 6.50.

10 ml of the rFVIIa/inhibitor solution was added to the column in aliquots of 0.5 ml and was collected in fractions of 0.5 ml.

The samples was analysed for content of inhibitor and rFVIIa by GPC. The relation between elution volume, inhibitor concentration and rFVIIa concentration is shown in Figure 6.

Figure 6 shows that within the tested elution volume (0.5 ml to 3 ml) a reduction of the inhibitor concentration to less than 175 microM is obtained (a reduction factor of approximately 10, as the initial value was 1,5 mM) and at the same time the rFVIIa concentration is unchanged compared to the initial value of 0,9 mg/ml.

## Claims

1. A method of removing a protein inhibitor from a liquid pharmaceutical preparation, said method comprising the steps of:

   (a) providing a sealed container comprising an initial liquid pharmaceutical preparation comprising (i) a protein belonging to the group of serine proteases/Vitamin K-dependent proteins and (ii) a non-metallic inhibitor for said protein;
   (b) contacting said pharmaceutical preparation with a solid phase polymeric material capable of retaining at least a substantial portion of said inhibitor: and
   (c) separating said liquid pharmaceutical preparation from said solid phase polymeric material so as to obtain a resulting liquid pharmaceutical preparation having a reduced concentration of said inhibitor compared to the initial liquid pharmaceutical preparation.

2. The method according to claim 1, wherein the protein has autocatalytic properties.

3. The method according to any one of the preceding claims, wherein the protein is in partly or fully activated form.

4. The method according to any one of the preceding claims, wherein the protein is selected from the group consisting of GAS-6, Protein S, Factor II (Prothrombin), Factor X polypeptides, Factor IX polypeptides, Protein C, Factor VII polypeptides, Protein Z, Transmembrane gamma-carboxyglutamic acid protein 1, Transmembrane gamma-carboxyglutamic acid protein 2, Transmembrane gamma carboxyglutamic acid protein 3, Transmembrane gamma-carboxyglutamic acid protein 4, Matrix Gla protein, and Osteocalcin.

5. The method according to any one of the preceding claims, wherein the protein is a Vitamin K-dependent coagulation factor selected from the group consisting of Factor VII polypeptides, Factor IX polypeptides, Factor X polypeptides and activated Protein C.

6. The method according to any one of the preceding claims, wherein the protein is a Factor VII polypeptide, preferably a Factor VII polypeptide in activated form.

7. The method according to any one of the preceding claims, wherein the inhibitor is a non-metallic compound having a molecular weight of at the most 1000 Da, e.g. at the most 750 Da, such as at the most 500 Da.

8. The method according to any one of the preceding claims, wherein the inhibitor is selected from the group consisting of amidines and guanidines.

9. The method according to any one of the preceding claims, wherein the protein is a Factor VII polypeptide and the inhibitor is selected from the group consisting of benzamidines and guanidines.

10. The method according to any one of the preceding claims, wherein the polymeric material is selected from the group consisting of cation-exchange materials, anion exchange materials, polar materials, non-polar materials, hydrophilic materials, and hydrophobic materials.

11. The method according to any one of the preceding claims, wherein the polymeric material is selected from the group consisting of cellulose fibres and hydrophilic synthetic polymers.

12. The method according to any one of the preceding claims, wherein the polymeric material is selected from the group consisting of cation-exchange materials.

13. The method according to any one of the preceding claims, wherein the liquid pharmaceutical preparation is contacted with the polymeric material by passing the liquid pharmaceutical preparation through a bulk of the polymeric material.

14. The method according to any one of the claims 1-12, wherein the liquid pharmaceutical preparation is contacted with the polymeric material by passing the liquid pharmaceutical preparation over a bulk of the polymeric material.

15. The method according to any one of the preceding claims, wherein the amount of the pharmaceutical preparation in said sealed container corresponds to 1-10 doses, e.g. 1-5 doses, in particular 1 dose.

16. The method according to claim 15, wherein the amount of the pharmaceutical preparation in said sealed container corresponds to 1 dose, and the dose is passed through or is passed over a bulk of the polymeric material.

17. The method according to claim 15, wherein the amount of the pharmaceutical preparation in said sealed container corresponds to 2-10 doses, and an amount corresponding to one dose is passed through or is passed over a bulk of the polymeric material.

18. The method according to any one of the preceding claims, wherein the contact time in step (b) is in the range of 0.1-100 sec.

19. A method according to any one of the preceding claims, said method comprising the steps of:

(a) providing a sealed container comprising an initial liquid pharmaceutical preparation comprising a Factor VII polypeptide and an inhibitor selected from benzamidines and guanidines;
(b) contacting said liquid pharmaceutical preparation with a solid phase polymeric material capable of retaining at least a substantial portion of said inhibitor, said polymeric materials being selected from the group consisting of cellulose fibres and hydrophilic synthetic polymers; and
(c) separating said liquid pharmaceutical preparation from said solid phase polymeric material so as to obtain a resulting liquid pharmaceutical preparation having a reduced concentration of said inhibitor compared to the initial liquid pharmaceutical preparation.

20. The method according to claim 19, wherein the polymeric material is selected from the group consisting of cation-exchange materials.

**Patentansprüche**

1. Verfahren zum Entfernen eines Proteinhemmers aus einer flüssigen pharmazeutischen Zubereitung, wobei das Verfahren folgende Schritte umfasst:

(a) Bereitstellen eines dicht verschlossenen Behälters, umfassend eine flüssige pharmazeutische Ursprungszubereitung, umfassend (i) ein Protein, das zu der Gruppe der Serinproteasen/vitamin-K-abhängigen Proteine gehört und (ii) einen nichtmetallischen Hemmer des Proteins;
(b) Kontaktieren der pharmazeutischen Zubereitung mit einem polymeren Festphasenmaterial, das mindestens einen wesentlichen Anteil des Hemmers festhalten kann; und
(c) Trennen der flüssigen pharmazeutischen Zubereitung von dem polymeren Festphasenmaterial, um so eine flüssige pharmazeutische Zubereitung mit einer im Vergleich zur flüssigen pharmazeutischen Ursprungszubereitung geringeren Konzentration des Hemmers zu erhalten.

2. Verfahren nach Anspruch 1, wobei das Protein autokatalytische Eigenschaften aufweist.

3. Verfahren nach einem der vorhergehenden Ansprüche, wobei das Protein in teilweise oder vollständig aktivierter Form vorliegt.

4.  Verfahren nach einem der vorhergehenden Ansprüche, wobei das Protein ausgewählt ist aus der Gruppe, bestehend aus GAS-6, Protein S, Faktor II (Prothrombin), Faktor-X-Polypeptiden, Faktor-IX-Polypeptiden, Protein C, Faktor-VII-Polypeptiden, Protein Z, transmembranem gamma-Carboxyglutaminsäureprotein 1, transmembranem gamma-Carboxyglutaminsäureprotein 2, transmembranem gamma-Carboxyglutaminsäureprotein 3, transmembranem gamma-Carboxyglutaminsäureprotein 4, Matrix-Gla-Protein und Osteocalcin.

5.  Verfahren nach einem der vorhergehenden Ansprüche, wobei das Protein ein vitamin-K-abhängiger Koagulationsfaktor ist, ausgewählt aus der Gruppe, bestehend aus Faktor-VII-Polypeptiden, Faktor-IX-Polypeptiden, Faktor-X-Polypeptiden und aktiviertem Protein C.

6.  Verfahren nach einem der vorhergehenden Ansprüche, wobei das Protein ein Faktor-VII-Polypeptid, vorzugsweise ein Faktor-VII-Polypeptid in aktivierter Form, ist.

7.  Verfahren nach einem der vorhergehenden Ansprüche, wobei der Hemmer eine nichtmetallische Verbindung mit einem Molekulargewicht von höchstens 1000 Da, beispielsweise höchstens 750 Da, wie höchstens 500 Da, ist.

8.  Verfahren nach einem der vorhergehenden Ansprüche, wobei der Hemmer ausgewählt ist aus der Gruppe, bestehend aus Amidinen und Guanidinen.

9.  Verfahren nach einem der vorhergehenden Ansprüche, wobei das Protein ein Faktor-VII-Polypeptid ist und der Hemmer ausgewählt ist aus der Gruppe, bestehend aus Benzamidinen und Guanidinen.

10. Verfahren nach einem der vorhergehenden Ansprüche, wobei das polymere Material ausgewählt ist aus der Gruppe, bestehend aus Kationenaustauschermaterialien, Anionenaustauschermaterialien, polaren Materialien, nichtpolaren Materialien, hydrophilen Materialien und hydrophoben Materialien.

11. Verfahren nach einem der vorhergehenden Ansprüche, wobei das polymere Material ausgewählt ist der Gruppe, bestehend aus Zellulosefasern und hydrophilen synthetischen Polymeren.

12. Verfahren nach einem der vorhergehenden Ansprüche, wobei das polymere Material ausgewählt ist aus der Gruppe, bestehend aus Kationenaustauschermaterialien.

13. Verfahren nach einem der vorhergehenden Ansprüche, wobei die flüssige pharmazeutische Zubereitung mit dem polymeren Material kontaktiert wird, indem die flüssige pharmazeutische Zubereitung durch eine lose Masse aus polymerem Material geschickt wird.

14. Verfahren nach einem der Ansprüche 1-12, wobei die flüssige pharmazeutische Zubereitung mit dem polymeren Material kontaktiert wird, indem die flüssige pharmazeutische Zubereitung über eine lose Masse aus polymerem Material geschickt wird.

15. Verfahren nach einem der vorhergehenden Ansprüche, wobei die Menge an pharmazeutischer Zubereitung in dem dicht verschlossenen Behälter 1-10 Dosen, beispielsweise 1-5 Dosen, insbesondere 1 Dosis, entspricht.

16. Verfahren nach Anspruch 15, wobei die Menge an pharmazeutischer Zubereitung in dem dicht verschlossenen Behälter 1 Dosis entspricht und die Dosis durch oder über eine lose Masse aus polymerem Material geschickt wird.

17. Verfahren nach Anspruch 15, wobei die Menge an pharmazeutischer Zubereitung in dem dicht verschlossenen Behälter 2-10 Dosen entspricht und eine Menge, die 1 Dosis entspricht, durch oder über eine lose Masse aus polymerem Material geschickt wird.

18. Verfahren nach einem der vorhergehenden Ansprüche, wobei die Kontaktierzeit in Schritt (b) im Bereich 0,1-100 s liegt.

19. Verfahren nach einem der vorhergehenden Ansprüche, wobei das Verfahren die folgenden Schritte umfassend:

    (a) Bereitstellen eines dicht verschlossenen Behälters, umfassend eine flüssige pharmazeutische Ursprungszubereitung, umfassend ein Faktor-VII-Polypeptid und einen Hemmer, ausgewählt aus Benzamidinen und Guanidinen;

(b) Kontaktieren der pharmazeutischen Zubereitung mit einem polymeren Festphasenmaterial, das mindestens einen wesentlichen Anteil des Hemmers festhalten kann, wobei die polymere Materialien ausgewählt sind aus der Gruppe, bestehend aus Zellulosefasern und hydrophilen synthetischen Polymeren; und

(c) Trennen der flüssigen pharmazeutischen Zubereitung von dem polymeren Festphasenmaterial, um so eine flüssige pharmazeutische Zubereitung mit einer im Vergleich zur flüssigen pharmazeutischen Ursprungszubereitung geringeren Konzentration des Hemmers zu erhalten.

**20.** Verfahren nach Anspruch 19, wobei das polymere Material ausgewählt ist aus der Gruppe, bestehend aus Kationenaustauschermaterialien.

## Revendications

**1.** Méthode d'extraction d'un inhibiteur de protéine d'une préparation pharmaceutique liquide, ladite méthode comprenant les étapes suivantes :

(a) apport d'un récipient hermétiquement fermé contenant une préparation pharmaceutique liquide initiale contenant (i) une protéine appartenant au groupe des sérine protéases/protéines dépendantes de la vitamine K et (ii) un inhibiteur non métallique de ladite protéine ;

(b) mise en contact de ladite préparation pharmaceutique avec un matériau polymérique en phase solide capable de retenir au moins une partie substantielle dudit inhibiteur ; et

(c) séparation de ladite préparation pharmaceutique liquide dudit matériau polymérique en phase solide de manière à obtenir une préparation pharmaceutique liquide résultante possédant une concentration réduite dudit inhibiteur par comparaison à la préparation pharmaceutique liquide initiale.

**2.** La méthode selon la revendication 1, dans laquelle la protéine possède des propriétés autocatalytiques.

**3.** La méthode selon l'une quelconque des revendications précédentes, dans laquelle la protéine se trouve sous une forme partiellement ou totalement activée.

**4.** La méthode selon l'une quelconque des revendications précédentes, dans laquelle la protéine est choisie dans le groupe consistant en GAS-6, protéine S, facteur II (prothrombine), polypeptides du facteur X, polypeptides du facteur IX, protéine C, polypeptides du facteur VII, protéine Z, protéine à résidus d'acide gamma-carboxyglutamique transmembranaire 1, protéine à résidus d'acide gamma-carboxyglutamique transmembranaire 2, protéine à résidus d'acide gamma-carboxyglutamique transmembranaire 3, protéine à résidus d'acide gamma-carboxyglutamique transmembranaire 4, protéine Gla de la matrice et ostéocalcine.

**5.** La méthode selon l'une quelconque des revendications précédentes, dans laquelle la protéine est un facteur de coagulation dépendant de la vitamine K choisi dans le groupe consistant en polypeptides du facteur VII, polypeptides du facteur IX, polypeptides du facteur X et protéine C activée.

**6.** La méthode selon l'une quelconque des revendications précédentes, dans laquelle la protéine est un polypeptide du facteur VII, de préférence un polypeptide du facteur VII sous forme activée.

**7.** La méthode selon l'une quelconque des revendications précédentes, dans laquelle l'inhibiteur est un composé non métallique possédant une masse moléculaire de 1 000 Da au plus, par exemple 750 Da au plus, telle que 500 Da au plus.

**8.** La méthode selon l'une quelconque des revendications précédentes, dans laquelle l'inhibiteur est choisi dans le groupe consistant en amidines et guanidines.

**9.** La méthode selon l'une quelconque des revendications précédentes, dans laquelle la protéine est un polypeptide du facteur VII et l'inhibiteur est choisi dans le groupe consistant en benzamidines et guanidines.

**10.** La méthode selon l'une quelconque des revendications précédentes, dans laquelle le matériau polymérique est choisi dans le groupe consistant en matériaux échangeurs de cations, matériaux échangeurs d'anions, matériaux polaires, matériaux non polaires, matériaux hydrophiles et matériaux hydrophobes.

**11.** La méthode selon l'une quelconque des revendications précédentes, dans laquelle le matériau polymérique est choisi dans le groupe consistant en fibres de cellulose et polymères synthétiques hydrophiles.

**12.** La méthode selon l'une quelconque des revendications précédentes, dans laquelle le matériau polymérique est choisi dans le groupe consistant en matériaux échangeurs de cations.

**13.** La méthode selon l'une quelconque des revendications précédentes, dans laquelle la préparation pharmaceutique liquide est mise en contact avec le matériau polymérique en faisant passer la préparation pharmaceutique liquide au travers d'une masse du matériau polymérique.

**14.** La méthode selon l'une quelconque des revendications 1 à 12, dans laquelle la préparation pharmaceutique liquide est mise en contact avec le matériau polymérique en faisant passer la préparation pharmaceutique liquide sur une masse du matériau polymérique.

**15.** La méthode selon l'une quelconque des revendications précédentes, dans laquelle la quantité de préparation pharmaceutique dans ledit récipient hermétiquement fermé correspond à 1 à 10 doses, par exemple 1 à 5 doses, en particulier 1 dose.

**16.** La méthode selon la revendication 15, dans laquelle la quantité de préparation pharmaceutique dans ledit récipient hermétiquement fermé correspond à 1 dose, et on fait passer la dose au travers ou sur une masse du matériau polymérique.

**17.** La méthode selon la revendication 15, dans laquelle la quantité de préparation pharmaceutique dans ledit récipient hermétiquement fermé correspond à 2 à 10 doses, et on fait passer une quantité correspondant à 1 dose au travers ou sur une masse du matériau polymérique.

**18.** La méthode selon l'une quelconque des revendications précédentes, dans laquelle le temps de contact de l'étape (b) se situe dans la plage de 0,1 à 100 secondes.

**19.** Méthode selon l'une quelconque des revendications précédentes, ladite méthode comprenant les étapes suivantes :

(a) apport d'un récipient hermétiquement fermé contenant une préparation pharmaceutique liquide initiale contenant un polypeptide du facteur VII et un inhibiteur choisi parmi les benzamidines et les guanidines ;
(b) mise en contact de ladite préparation pharmaceutique liquide avec un matériau polymérique en phase solide capable de retenir au moins une partie substantielle dudit inhibiteur, ledit matériau polymérique étant choisi dans le groupe consistant en fibres de cellulose et polymères synthétiques hydrophiles ; et
(c) séparation de ladite préparation pharmaceutique liquide dudit matériau polymérique en phase solide de manière à obtenir une préparation pharmaceutique liquide résultante possédant une concentration réduite dudit inhibiteur par comparaison à la préparation pharmaceutique liquide initiale.

**20.** La méthode selon la revendication 19, dans laquelle le matériau polymérique est choisi dans le groupe consistant en matériaux échangeurs de cations.

## Removal of inhibitor* by Chelex 100

*S-2-[3-(4-carbamimidoylphenyl)-ureido]-N-[1-(3-methoxyphenyl)-ethyl]-acetamide

## Fig. 1

## Removal of p-aminoBenzamidine by Chelex 100

## Fig. 2

Fig. 3a and 3b

Fig. 4a and 4b

**Fig. 5**

**Fig. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9715391 A
- WO 2005016365 A
- US 4784950 A
- WO 0179271 A
- WO 0331464 A
- US 20040043446 A
- US 20040063911 A
- US 20040142856 A
- US 20040137557 A
- US 20040132640 A
- WO 0104287 A
- US 20030165996 A
- WO 0158935 A
- WO 0393465 A
- WO 0202764 A
- US 20030211094 A
- US 5580560 A
- DK 0200189 W
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- US 6806063 B
- US 20030096338 A
- WO 04029091 A
- WO 04083361 A
- WO 04111242 A
- WO 04108763 A
- WO 0183725 A
- WO 0222776 A
- DK 0200635 W
- WO 03027147 A
- DK PA200201423
- WO 04029090 A
- DK PA200101627
- WO 0524006 A
- WO 05123916 A
- JP 2001061479 B
- EP 1162194 A1
- EP 1270551 A1
- WO 9215686 A
- US 5997864 A

### Non-patent literature cited in the description

- *Eur. J. Biochem.,* 2001, vol. 268, 1554-1560
- *Bioorg. Med. Chem. Lett.,* 2003, vol. 13, 1463-1467
- **Rannels et al.** *Proc. Natl. Acad. Sci. USA,* 1987, vol. 84, 5952-56
- **Lino et al.** *Arch. Biochem. Biophys.,* 1998, vol. 352, 182-192
- **Mollerup et al.** *Biotechnol. Bioeng.,* 1995, vol. 48, 501-505
- **Kornfelt et al.** *Arch. Biochem. Biophys.,* 1999, vol. 363, 43-54
- **Persson et al.** *J. Biol. Chem.,* 1997, vol. 272, 19919-19924
- **Persson.** *FEBS Letts,* 1997, vol. 413, 359-363
- **Charles M. Hansen.** Hansen's Solubility Parameters: A User's Handbook. CRC Press, 1999
- **Monroe et al.** *Brit. J. Haematol.,* 1997, vol. 99, 542-547